# EUROPEAN PATENT APPLICATION

(11) **EP 2 774 970 A1**
(43) Date of publication of application: **10.09.2014**
(21) Application number: 14158308.8
(22) Date of filing: 07.03.2014
(51) Int. Cl.: C10G 1/00, C10G 27/04, C10G 27/00, C10G 27/14, C07C 1/207, C10L 1/02, B01J 32/00

(54) **Process for converting bio-oil**

(30) Priority: 08.03.2013 FI 20135224
(71) Applicant: UPM-Kymmene Corporation, 00100 Helsinki (FI)
(72) Inventor: Asikkala, Janne, 53950 Lappeenranta (FI); Gutierrez, Andrea, 53200 Lappeenranta (FI); Kotilainen, Risto, 45100 Kouvola (FI)
(74) Representative: Boco IP Oy Ab

(57) **Abstract**

The present invention relates to a process for converting bio-oil, said process comprising the steps, where feedstock comprising bio-oil, which is selected from bio-oils and any fractions of bio-oils and any combinations thereof, is subjected to oxidation in the presence of an oxidant, under conditions suitable for enacting said oxidation to yield an oxidation product and subjecting said oxidation product to condensation in the presence of a basic catalyst to obtain converted bio-oil. The invention also relates to the use of converted bio-oil, obtainable by said process, as heating oil, as starting material in processes for producing fuels, fuel components, fine chemicals, chemical building-blocks, and solvents.

## Description

### FIELD OF THE INVENTION

The present invention relates to converting of bio-oil, whereby the composition of the bio-oil is altered, acidity is decreased and the stability of the bio-oil is improved. The invention also relates to subjecting bio-oil to oxidation under conditions suitable for oxidation to yield an oxidation product and subjecting said oxidation product to condensation under conditions suitable for condensation to obtain converted bio-oil. The invention also relates to converted bio-oils obtainable by said process.

### BACKGROUND OF THE INVENTION

Bio-oils of varying properties and compositions are obtained using numerous methods and processes. Bio-oils may be obtained for example from biomass using any suitable thermal treatment, pyrolysis and the like.

Pyrolysis is generally understood as the chemical decomposition of organic materials by heating in the absence or with limited supply of oxidizing agent such as air or oxygen. Pyrolysis can be used for converting biomass to pyrolysis oil which is an example of bio-oil. Commercial pyrolysis applications are typically either focused on the production of charcoal (slow pyrolysis) or production of liquid products (fast pyrolysis), the pyrolysis oil. Both the slow pyrolysis and the fast pyrolysis processes may be used for the manufacture of pyrolysis oil.

During pyrolysis of biomass, for example of lignocellulosic material, carried out at temperatures in the range 400-700°C, most of the cellulose and hemicellulose and part of lignin typically disintegrate to form smaller and lighter molecules which are vapors at the pyrolysis temperatures. During cooling some of the vapors condense to form a liquid product, called pyrolysis oil.

Bio-oils are complex mixtures of chemical compounds, including reactive aldehydes and ketones. Said reactive compounds react with each other whereby complex molecules having higher molecular weight are formed and the viscosity of bio-oil is increased. For example biomass derived pyrolysis oil typically comprises water, light volatiles and non-volatiles. Further, pyrolysis oil has high acidity, which typically leads to corrosion problems, substantial water content, and high oxygen content. Wood-based pyrolysis oil is the product of pyrolysis of wood or forest residues and it contains typically carboxylic acids, aldehydes, ketones, carbohydrates, thermally degraded lignin, water, and alkali metals. The oxygen-containing compounds (typically 40-50 wt-%) and water (typically 15-30 wt-%) make pyrolysis oils chemically and physically unstable. Although pyrolysis oils have higher energy density than wood, they are acidic (pH∼2) and incompatible with conventional fuels. Furthermore pyrolysis oils have high viscosity and high solid content. Poor stability and high acidity are one of the key problems in utilizing the pyrolysis oil or storing for longer periods.

Due to its instability bio-oil is rapidly transformed to semisolid and gradually solid material, which is difficult to store or use for any further purposes. Thus, according to present practice it is necessary to process the bio-oils rapidly further in order to avoid the problems relating to stability.

Refining of bio-oils and particularly pyrolysis oil to provide fuel or fuel components is often very challenging due to the complex mixture of components of said bio-oil. For example pyrolysis oil typically consists of more than 200 identified compounds, which require very different conditions for converting them further to fuel components or precursors to fuel. Often this is carried out by hydroprocessing said pyrolysis oil over a hydrogenation catalyst in the presence of hydrogen. Since pyrolysis oil typically contains up to 50 wt% of oxygen, complete removal oxygen requires a substantial amount of hydrogen, even up to 1000 L/kg pyrolysis oil. The obtained light components are turned into gaseous products (hydrogen, methane, ethane, etc.) and heavy components are turned into coke and heavy oil. The heavy oil mixture needs further refinement to produce fuel fractions and this procedure requires high amounts of hydrogen and typically various different catalysts for obtaining the desired products.

Despite the ongoing research and development relating to bio-oils, there is still a need to provide improved processes and methods for converting bio-oils to more valuable components in an efficient and economical way.

### SUMMARY OF THE INVENTION

The present invention relates a process for converting bio-oil, whereby the composition of said bio-oil is altered, acidity is decreased and stability of the bio-oil is improved. Particularly the present invention relates to a process for converting bio-oil, where feedstock comprising bio-oil is subjected to oxidation under conditions suitable for oxidation to yield an oxidation product and subjecting said oxidation product to condensation under conditions suitable for condensation to obtain converted bio-oil. In the process converted bio-oil, having improved stability and less complicated composition may be obtained, whereby the converted bio-oil is maintained in liquid form for long periods of time.

The present invention also provides converted bio-oil, which may be used as such as heating oil and as starting material in processes for producing fuels, fuel components, fine chemicals and chemical building-blocks for chemical production and solvents.

The process for converting bio-oil comprises the steps where a feedstock comprising bio-oil is subjected to oxidation in the presence of an oxidant selected from O₂, O₃ and H₂O₂, under conditions suitable for enacting said oxidation to yield an oxidation product and subjecting said oxidation product to condensation in the presence of a basic catalyst to obtain converted bio-oil. The term "basic catalyst" includes here alkaline catalysts.

Thus an object of the invention is to provide a process for effectively and economically converting bio-oil, whereby the composition of said bio-oil is altered, viscosity is decreased and stability improved.

Another object of the invention is to provide converted bio-oil, suitable for use as such or in the manufacture of valuable components, particularly fuels and fuel components.

Still another object of the invention is to provide converted bio-oils based at least partly or totally on renewable starting materials for use as such or in the manufacture of valuable components.

### DEFINITIONS

The term "hydroprocessing" refers here to catalytic processing of organic material by all means of molecular hydrogen.

The term "carbonyl compounds" refers here to all organic molecules containing one or more carbonyl groups, particularly aldehydes and ketones.

The term "chemical building-blocks" or "building-block chemicals" refer to chemical compounds useful as starting materials and intermediates for the manufacture of chemical and pharmaceutical final products. Examples of such chemical building-blocks are fumaric acid, furfural, glycerol, citric acid, treonin, propanic acid etc.

Transportation fuels refer to fractions or cuts or blends of hydrocarbons having distillation curves standardized for fuels, such as for diesel fuel (middle distillate from 160 to 380°C, EN 590), gasoline (150 - 210°C, EN 228), aviation fuel (160 to 300°C, ASTM D-1655 jet fuel), kerosene, naphtha, etc.

### BRIEF DESCRIPTION OF THE DRAWINGS

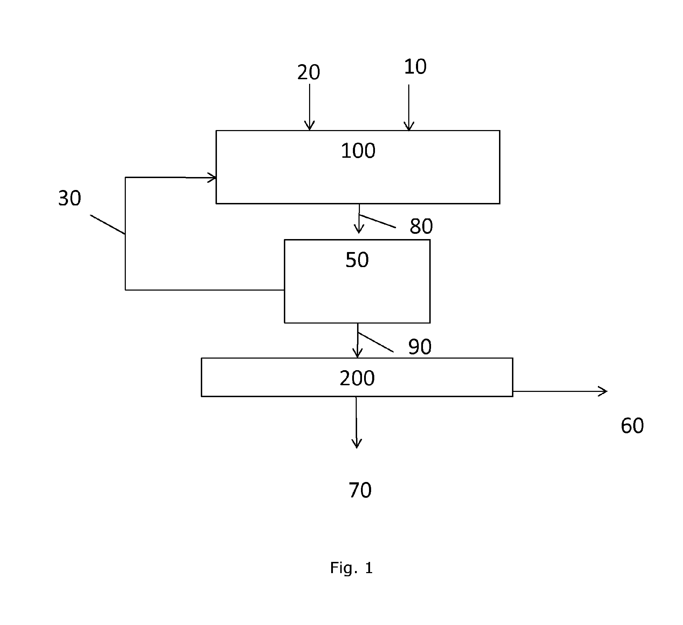

Fig. 1 is a schematic flow diagram representing one embodiment of the process for converting bio-oils.

### DETAILED DESCRIPTION OF THE INVENTION

It was surprisingly found that a feedstock comprising bio-oils can be converted in an efficient manner to valuable products, with a process where oxidation and condensation of the oxidation product are carried out. In said process a feedstock comprising bio-oil is subjected to oxidation under conditions suitable for oxidation to yield an oxidation product and subjecting said oxidation product to condensation under conditions suitable for condensation to obtain converted bio-oil.

In the oxidation step organic molecules can be degraded, whereby the oxidant (oxidation agent) forms carboxylic acid functions in the organic molecules, and further the oxidation breaks C-C bonds and can depolymerize complex molecules. The oxidation products are carboxylic acids, which are then condensed into longer chain oxygen containing hydrocarbons, particularly alcohols and/or saturated carbon chain (see scheme 1). For example Aldol condensation may be utilized is step 2.

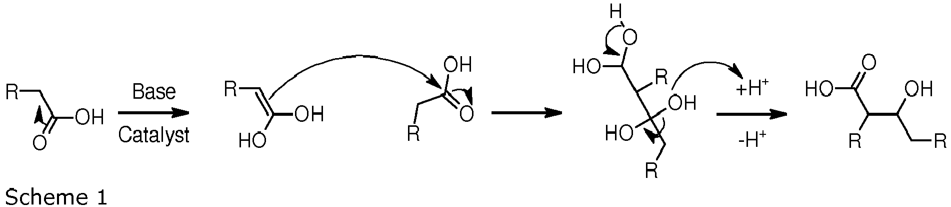

The oxidation step will produce more homogenous product from bio-oil and the condensation step increases the chain length of the compounds contained in the product.

The converted bio-oil may be used as starting material or feedstock in further refinement steps, as described for example in scheme 2 (hydrogenation), where the hydrogen consumption in the hydrogenation may be decreased significantly and more valuable long chain hydrocarbons may be obtained, particularly suitable as fuels or fuel components, such as transportation fuels.

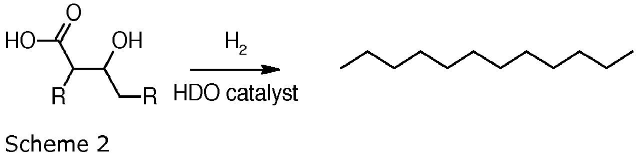

The process for converting bio-oil comprises the steps, where feedstock comprising bio-oil is subjected to oxidation in the presence of an oxidant selected from O₂, O₃ and H₂O₂, under conditions suitable for enacting said oxidation to yield an oxidation product and subjecting said oxidation product to condensation in the presence of a basic catalyst to obtain converted bio-oil.

Figure 1 is a schematic diagram of a process in accordance with one embodiment of the invention. In this embodiment, in the first step feedstock comprising bio-oil 10, oxidant 20 and solvent 30 are fed to a reactor 100 wherein oxidation is carried out. The oxidation reaction product 80 is subjected to separation in separation unit 50, where solvent 30 is separated and recycled to the oxidation reactor 100, and the liquid oxidation product 90 is directed to condensation reactor 200 where condensation is carried out, water 60 is separated from the reactor and converted liquid bio-oil 70 is obtained as the product.

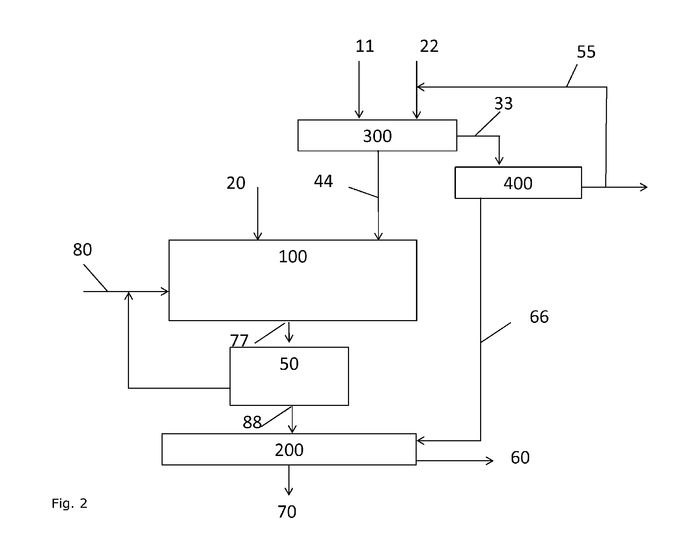

Figure 2 is a schematic diagram of a process in accordance with another embodiment of the invention. In this embodiment, bio-oil 11 is first treated with an aqueous media. Bio-oil 11 and aqueous media 22 are charged to reactor 300 where they are mixed and then separated into an aqueous phase 33 and into an organic (containing water insoluble fraction) phase 44. The aqueous phase 33 may be directed to further processing, purification and fractionation 400 for recovering valuable chemicals from it. The aqueous phase 33 may subjected to further fractionation to give an organic phase 66 and aqueous phase 55, where the organic phase 66 is recycled to the condensation reactor 200 and the separated aqueous phase 55 may be recycled to the aqueous media feed 22, and it may also be directed to oxidation reactor 100 (not shown in the figure). The organic phase 44, oxidant 20 and solvent 30 are fed to the oxidation reactor 100 wherein oxidation is carried out. The liquid reaction product is subjected to separation in separation unit 50, where solvent 80 is separated. The separated solvent 80 may be recycled to the oxidation reactor 100. From the separation unit 50 the liquid oxidation product 88 is directed to condensation reactor 200 where condensation is carried out, water 60 is separated from the reactor and converted liquid bio-oil 70 is obtained as the product.

The feedstock comprising bio-oil is selected from bio-oils, from any fractions of bio-oils and any combinations thereof. Bio-oil means here any oils or oily components obtained from any known thermal processing of biomass, from any supercritical fluid treatment of biomass, from molten salt treatment of biomass, from ionic liquid treatment of biomass. Suitably pyrolysis oils and any combinations thereof are used. Said pyrolysis oil may be obtained from any pyrolysis process of biomass, including slow pyrolysis, fast pyrolysis, catalytic pyrolysis and hydropyrolysis (catalytic fast pyrolysis in the presence of hydrogen).

Biomass may typically comprise virgin and waste materials of plant, animal and/or fish origin or microbiological origin, such as virgin wood, wood residues, forest residues, waste, municipal waste, industrial waste or by-products, agricultural waste or by-products (including also dung or manure), residues or by-products of the wood-processing industry, waste or by-products of the food industry, solid or semi-solid organic residues of anaerobic or aerobic digestion, such as residues from bio-gas production from lignocellulosic and/or municipal waste material, residues from bio-ethanol production process, and any combinations thereof. Biomass may include the groups of the following four categories: wood and wood residues, including sawmill and paper mill discards, municipal paper waste, agricultural residues, including corn stover (stalks and straw) and sugarcane bagasse, and dedicated energy crops, which are mostly composed of tall, woody grasses.

Suitably biomass is selected from non-edible sources such as non-edible wastes and non-edible plant materials. Particularly suitably said biomass comprises waste and by-products of the wood-processing industry such as slash, urban wood waste, lumber waste, wood chips, wood waste, sawdust, straw, firewood, wood materials, paper, by-products of the papermaking or timber processes, where the biomass (plant biomass) is composed of cellulose and hemicellulose, and lignin.

The pyrolysis oil refers particularly to a complex mixture of oxygen containing compounds (oxygenates), comprising typically water, light volatiles and non-volatiles. Pyrolysis oil is acidic, with a pH of 1.5- 3.8, and wood based pyrolysis oil typically has pH between 2 and 3. The exact composition of pyrolysis oil depends on the biomass source and processing conditions. Typically pyrolysis oil comprises 20-30 % of water, 22-36 % of suspended solids and pyrolitic lignin (including low molecular mass lignin and high molecular mass lignin), 8-12 % of hydroxyacetaldehyde, 3-8 % of levoglucosan, 4-8 % of acetic acid, 3-6 % of acetol, 1-2 % of cellubiosan, 1-2 % of glyoxal, 3-4 % of formaldehyde, and 3-6 % of formic acid by weight. Pyrolysis oil typically also comprises other ketones, aldehydes, alcohols, furans, pyranes, sugars, organic acids, lignin fragments, phenolics, extractives and small amounts of inorganics. The density of pyrolysis oil is approximately 1.2-1.3 kg/l and usually the water molecules which are split during pyrolysis stay bound within the complex pyrolysis liquid as an emulsion.

Optionally water-insoluble bio-oil fractions, suitably water-insoluble pyrolysis oil fractions may be used as feedstock for oxidation or as part of it. Said fractions are suitably obtained by subjecting the bio-oil, particularly pyrolysis oil, to treatment with an aqueous media, whereby the water-soluble components and excess water are are separated from the water-insoluble fraction, as described below. In this way pyrolysis oil can be fractionated or "dried". The water-insoluble fraction contains mainly lignin with high molar mass and low molar mass compounds.

The (organic) water-insoluble fraction is conducted to the oxidation step of the present process whereby the consumption of the oxidant is decreased. The aqueous phase (containing the water-soluble fraction) may be subjected to further fractionation to give an organic phase and aqueous phase, where the organic fraction comprising small molecular weight compounds and lignin is recycled to the condensation step and the separated aqueous phase may be recycled as the aqueous media. In this way the smaller molar mass compounds in the water soluble fraction can be preserved and only higher molar mass water insoluble fraction is oxidized. This also increases the overall yield of conversion in the case the converted bio-oil product is further processed to other products, such as fuel etc.

Suitably the fractionation of bio-oil is carried out as follows. An aqueous media (such as water) is added to the bio-oil (pyrolysis oil) whereby a mixture is formed. The amount of aqueous media is from 50 to 98 vol-%, suitably from 55 to 95 vol-%, particularly suitably from 75 to 90 vol-% and the amount of pyrolysis oil is from 2 to 50 vol-%, suitably from 5 to 45 vol-%, particularly suitably from 10 to 25 vol-%. The obtained mixture, suitably having a temperature from 10 to 80°C, particularly suitably from 20 to 60°C, is agitated, suitably for 5 to 60 min and then it is allowed to settle whereby phases are formed. The organic (less polar) phase containing water insoluble and water immiscible compounds and the aqueous (more polar) phase containing water soluble and water miscible compounds are separated. In this aqueous media treatment step the pyrolysis oil is dried, whereby the water content of the organic phase is lower than that of the pyrolysis oil. In the aqueous phase water soluble and water miscible organic compounds and inorganic salts (such as alkali metal salts) are removed from the bio-oil, particularly sugars such as galactose, glucose, mannose, arabinose and xylose; organic acids, also glucuronic acid and galacturonic acid, aldehydes, ketones and some phenols.

The aqueous media is selected from water, waste water streams, recirculated aqueous streams from the process or from another processes. Said aqueous media is suitably free of metals, alkali metals and solid particles. Suitably tap water or deionized water is used.

The organic phase contains organic compounds insoluble or immiscible in water, such as phenolics and lignin residues.

The oxidant (oxidizing agent) is selected from O₂, O₃ and H₂O₂. If O₂ is used, at least one catalyst is needed. Said catalyst comprises a metal selected from Groups 3 -11 of the Periodic table of elements, suitably Al, Bi, Ce, Cd, Co, Cr, Cu, Fe, Mn, Ni, Ti, V, Y, Zn, Zr. Suitably salts of said metals, such as chlorides, bromides, fluorides, carboxylates, and the like or simple oxides such as CuO, CoO, Cr₂O₃, NiO or mixed oxides MnO₂-CeO₂, ZrₓCe₁₋ₓO₂ or supported noble metal catalyst comprising 0,1-0,5 wt% of Ir, Pd, Pt, Rh, Ru on Al₂O₃, CeO₂, TiO₂, ZrO₂ or SiO may be used. O₃ may be used without catalyst. H₂O₂ is typically used as a water solution (30 wt%) without catalyst. If desired a catalyst may be used to accelerate the oxidation reaction with both O₃ and H₂O₂.

The amount of the oxidant is suitably 0.01-10 kg/kg of the feedstock, particularly suitably 0.05-4 kg/kg of the feedstock.

Optionally at least one polar solvent selected from aqueous media as defined above, acetic acid and mixtures thereof may be used in the oxidation step in amounts providing the reaction mixture a suitable viscosity for carrying out the reaction.

The oxidation reaction is carried out at a temperature from 20 to 200°C, suitably from 80 to 150°C.

The oxidation reaction is carried out under a pressure from normal atmospheric pressure NTP to 40 bar, suitably from 5 to 20 bar, particularly when O₂, O₃ is used.

The oxidation reaction is suitably allowed to proceed for 1 to 50 h, particularly suitably from 3 to 20 h.

The obtained oxidation product (referring here also to the reaction mixture obtained from the oxidation reaction) may directly be transferred without any purification or separation steps to the condensation step, or optionally one or more separation and purification steps may be carried out prior to the condensation reaction step. Any suitable mean for separation may be used, such as cyclonic separation, distillation, scrubbers including amine scrubbers and the like.

After the oxidation reaction, solvent may be separated using any suitable means, such as evaporation, separation using a polar organic solvent, such as ethyl acetate, chlorinated solvents, methyl-tert-butylether etc. If H₂O₂ was used, unreacted H₂O₂ is destroyed with reagents known as such, for example Fe(II)SO₄*7H₂O prior to solvent separation.

The oxidation product is subjected to condensation in the presence of a basic catalyst. Said basic catalyst is selected from silicates, aluminates, zeolites, alkalimetal hydroxides, alkaline earth oxides, alkali metal oxides, rare earth oxides. Suitably ThO₂, ZrO₂, ZnO₂ and TiO₂, alkali ion-exchanged zeolites, alkali ion-added zeolites, alkali metal ions on alumina, alkali metal ions on silica, alkali metals on alkaline earth oxides, alkali metals and alkali metal hydroxides on alumina, on hydrotalcite, on chrysotile, on sepiolite, KF supported on alumina, lanthanide imide and nitride on zeolite may be used.

The following catalysts may also be used in the condensation step. Aldol additions and condensations are catalysed by Ba(OH)₂. Alkaline earth oxides, La₂O3, and Zr02 are also active for the reaction in the following order: BaO > SrO>CaO > MgO>, La203 > Zr02. Zeolites are also active in aldol additions and condensations.

The condensation reaction is carried out at a temperature from 300 to 450°C, suitably from 350 to 400°C.

The condensation reaction is carried out under a pressure from NTP to 20 bar, suitably from 5 to 15 bar.

After the condensation reaction, different fractions of the converted bio-oil product may be separated by fractionation based on boiling point (distillation), such as into light and heavy fraction. The fractions may not have desired quality (gasoline, diesel etc.) and further processing may be required. These further processing could be e.g. hydroprocessing steps, such as hydrogenation, hydrodeoxygenation on conventional hydrotreating catalysts.

The process may be carried out a batch process, semi-batch process or a continuous process. In the process and in the oxidation and condensation steps any suitable reactors, equipment and configurations may be used, suitable for handling materials which may be corrosive. For example any conventional reactors, tubular reactors, plug flow reactor as well as packed reactors, slurry reactors and fluid-bed reactors may be used.

An oily, liquid converted bio-oil product is obtained having less acidity, lower amount of acids, lower amount of oxygen containing compounds, decreased viscosity, and it is a less complicated mixture of compounds. It has clearly increased stability and it is less corrosive.

With the process bio-oils and particularly pyrolysis oils can be upgraded in an effective and economic way.

The converted bio-oil product may be used as such for heating purposes as heating oil, where it provides clear advantages, such as higher heating value and higher quality than that of conventional bio-oils, such as pyrolysis oils. Due the improved stability and quality it may also be used as starting material in wider range of processes including processes for producing fuels, fuel components, particularly transportation fuels, fine chemicals and chemical building-blocks for chemical production, and solvents.

If desired the converted bio-oil product may be subjected to any known hydroprocessing steps, and any pretreatment and purification steps. Particularly in hydroprocessing simple hydrogenation conditions are sufficient and no complicated measures are needed, the consumption of H₂ is lower due to lower O₂ content in the converted bio-oil product, the yield are increased and better control of products is achieved.

Further, if water-insoluble fractions of bio-oils are used as feedstock for the oxidation, the oxidation of small molecular weight compounds to CO₂ can be prevented, oxidation can be controlled better, acids removed in the water-soluble fraction can be utilized in the condensation step and yields are further improved.

It was surprisingly found that the feedstock (pyrolysis oil) can be converted into a water soluble bio-oil product. In the bio-oil product the molecules in the oxygenated compounds are smaller than in feed pyrolysis oil (no more large polymers e.g. lignin). Significant advantages are thus achieved particularly with respect to further uses and processing of the converted bio-oil.

The following examples are illustrative of embodiments of the present invention, as described above, and they are not meant to limit the invention in any way. The invention is illustrated also with reference to the drawings.

### Examples

The elemental composition of the pyrolysis oil used as feedstock in examples 1 and 2 is provided in table 1 below.

**Table 1. Composition of the pyrolysis oil**

| C | H | N | S | O | Density (g/cm³) | Water content |
|---|---|---|---|---|---|---|
| 41.53 | 5.92 | 0.19 | Not detected | 34.68 | 1.16 | 30.8 wt% |

### Example 1

### Oxidation of pyrolysis oil with hydrogen peroxide

Pyrolysis oil (50 g) was dissolved in 100 ml water containing 30 % H₂O₂. The reaction mixture was slowly heated to 100°C and water started to reflux. The reaction was continued for 3 hours at refluxing water. Fe(II)SO₄*7H₂O was added to the reaction mixture to remove all unreacted H₂O₂. After all peroxide was removed, the reaction mixture was evaporated under reduced pressure to yield dark brown mixture.

GC-MS revealed alcohols (2-methyl-4-oxo-pentan-2-ol, glycerol), carboxylic acids (acetic acid, propenoic acids) and dicarboxylic acids (succinic acid, malonic acid).

The reaction mixture is subjected to condensation whereby a bio-oil product is obtained having reduced content of small molecular weight carboxylic acids and decreased acid number.

### Example 2

### Fractionation and oxidation of pyrolysis oil with hydrogen peroxide

Wood based pyrolysis oil (25 ml) was fractionated by adding 25 ml of water. After water addition the mixture was mixed with vortex for about 10 min and then centrifuged at 2500 rpm for 15 min. This allowed the separation of water soluble and organic fraction of pyrolysis oil. The organic fraction of pyrolysis oil was oxidized. 31 g was mixed with 100 ml water containing 30 % H₂O₂. The reaction mixture was slowly heated to 100°C and water started to reflux. The reaction was continued for 23 hours at refluxing water. Fe(II)SO₄*7H₂O was added to the reaction mixture to remove all unreacted H₂O₂. After all peroxide was removed, the reaction mixture was extracted with methyl-terbutyl ether (MTBE) to remove the organic soluble material (yield was 40 %). The water soluble carboxylic acid fraction was evaporated under reduced pressure to yield dark brown mixture (yield about 90%).

**Table 2. Elemental composition of MTBE phase of oxidized pyrolysis oil.**

| C | H | N | S* | O |
|---|---|---|---|---|
| 44,08 | 6,61 | 0,08 | Not measured | 30,91 |

The sulfur content is almost negligible in wood-based pyrolysis oils.

GC-MS revieled that the MTBE phase contains alcohols (2-methyl-4-oxo-pentan-2-ol) carboxylic acids (acetic acid, 4-oxopentanoic acid, 4-oxohexanoic acid, vanillic acid, 4-hydoxybenzoic acid), dicarboxylic acids (Butanedienoic acid, 3-methyl-1,5-pentanedienoic acid, 1,6-hexanedienoic acid, 1,9-nonadienoic acid).

The carboxylic acid fraction is subjected to condensation whereby a bio-oil product is obtained having reduced content of small molecular weight carboxylic acids and decreased acid number.

The present invention has been described herein with reference to specific embodiments. It is, however clear to those skilled in the art that the process(es) may be varied within the bounds of the claims.

## Claims

1. A process for converting bio-oil, wherein said process comprises the steps where a feedstock comprising bio-oil is subjected to oxidation in the presence of an oxidant to yield an oxidation product and subjecting said oxidation product to condensation carried out in the presence of a basic catalyst to obtain converted bio-oil.

2. The process according to claim 1, wherein the bio-oil is selected from oils or oily components obtained from thermal processing of biomass, from supercritical fluid treatment of biomass, from molten salt treatment of biomass, from ionic liquid treatment of biomass, pyrolysis oils, and fractions of bio-oils, and combinations thereof.

3. The process according to claim 2, wherein the pyrolysis oil is subjected to treatment with an aqueous media to obtain a water-soluble fraction and water-insoluble fraction and the water-insoluble fraction is subjected to the oxidation.

4. The process according to any one of claims 1-3, wherein at least one polar solvent is used in the oxidation and after the oxidation said solvent is separated from the oxidation product and recycled to the oxidation.

5. The process according to claim 4, wherein the polar solvent is selected from aqueous media, acetic acid and mixtures thereof.

6. The process according to any one of claims 3-5, wherein the water soluble fraction is subjected to fractionation to give an organic phase and aqueous phase, and the organic phase is recycled to the condensation and the aqueous phase is recycled to the aqueous media.

7. The process according to any one of claims 1-6, wherein the oxidant is selected from O₂, O₃ and H₂O₂.

8. The process according to any one of claims 1-7, wherein a catalyst comprising a metal selected from Groups 3-11 of the Periodic table of elements is used in the oxidation.

9. The process according to any one of claims 1-8, wherein oxidation is carried out at a temperature from 20 to 200°C, preferably from 80 to 150°C.

10. The process according to any one of claims 1-9, wherein the oxidation is carried out under a pressure from normal atmospheric pressure NTP to 40 bar.

11. The process according to any one of claims 1-10, wherein the oxidation is carried out under a pressure from 5 to 20 bar.

12. The process according to any one of claims 1-11, wherein the basic catalyst is selected from silicates, aluminates, zeolites, alkalimetal hydroxides alkaline earth oxides, alkali metal oxides, rare earth oxides, preferably Th02, Zr02, Zn02, Ti02, alkali ion-exchanged zeolites, alkali ion-added zeolites, alkali metal ions on alumina, alkali metal ions on silica, alkali metals on alkaline earth oxides, alkali metals and alkali metal hydroxides on alumina, on hydrotalcite, on chrysotile, on sepiolite, KF supported on alumina, lanthanide imide and nitride on zeolite.

13. The process according to any one of claims 1-12, wherein the condensation is carried out at a temperature from 300 to 450°C.

14. The process according to any one of claims 1-13, wherein the condensation is carried out under a pressure from NTP to 20 bar.

15. Use of the converted bio-oil product obtainable from the process according to any one of claims 1-14 as heating oil, as starting material in processes for producing fuels, fuel components, fine chemicals, chemical building-blocks, and solvents.
